# FASCICULE DE BREVET EUROPEEN

(11) **EP 2 496 242 B1**
(45) Date de publication et mention de la délivrance du brevet: **27.06.2018**
(21) Numéro de dépôt: 10787152.7
(22) Date de dépôt: 02.11.2010
(51) Int. Cl.: A61K 36/064, A61K 8/99, A61Q 5/00, A61Q 7/00, A61P 17/14

(54) **UTILISATION D'UN HYDROLYSAT PEPTIDIQUE DE LEVURE EN TANT QU'AGENT ACTIF POUR RENFORCER LE CHEVEU**
VERWENDUNG EINES HEFEPEPTIDHYDROLYSATS ALS WIRKSTOFF ZUR HAARSTÄRKUNG
USE OF YEAST PEPTIDE HYDROLYSATE AS AN ACTIVE AGENT FOR STRENGTHENING HAIR

(30) Priorité: 03.11.2009 FR 0905257
(43) Date de publication de la demande: 12.09.2012
(73) Titulaire: ISP Investments Inc., Wilmington, DE 19805 (US)
(72) Inventeur: DAL FARRA, Claude, Kerhonkson NY 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2010/000724
(87) Numéro de publication internationale: WO 2011/055034

(56) Documents cités:
- EP-A2- 0 695 801
- FR-A1- 2 904 552
- FR-A1- 2 927 254

## Description

### Domaine de l'invention

La présente invention se situe dans le domaine cosmétique. L'invention concerne l'utilisation d'une composition comprenant au moins un hydrolysat peptidique de levure (*Saccharomyces cerevisiae)*, en tant qu'agent actif, pour renforcer le cheveu et améliorer la santé du cheveu.

L'invention porte encore sur un procédé de traitement cosmétique destiné à stimuler la pousse ou lutter contre la chute du cheveu et à lutter contre les agressions extérieures touchant le cheveu, selon lequel on applique topiquement une composition contenant l'agent actif sur les zones à traiter.

### Arrière plan de l'invention

La croissance des cheveux et leur renouvellement sont principalement déterminés par l'activité des follicules de cheveu et de leur environnement matriciel.

Le follicule de cheveu est une annexe cutanée autonome complexe qui comporte six grands compartiments, les uns d'origine dermique (gaine conjonctive et papille dermique), les autres de nature épithéliale (gaines épithéliales interne et externe, tige pilaire et sébacée). A la base du follicule se trouve la matrice, richement vascularisée, à l'origine des 3 couches concentriques du cheveu. Le cheveu est constitué de cellules épithéliales différenciées contenant essentiellement des kératines, elles-mêmes organisées en superstructures protéiques très stables. L'organisation et la solidité de la tige pilaire et du cheveu sont déterminées par l'activité et la bonne santé du follicule.

Le follicule de cheveu se renouvelle selon un cycle d'activité qui comprend trois phases : la phase anagène, la phase catagène et la phase télogène.

La phase anagène (phase de croissance) dure de un à dix ans et se caractérise par l'allongement constant du cheveu. Au début de chaque phase anagène, on observe un développement du micro-réseau vasculaire périfolliculaire. La microcirculation périfolliculaire joue donc un rôle primordial dans le processus de croissance du cheveu, en apportant les facteurs et les nutriments nécessaires à la croissance du follicule.

La phase catagène qui succède dure seulement quelques semaines. Au cours de cette phase, les capillaires sanguins se collapsent et disparaissent. Le follicule s'atrophie et se rétracte vers la surface, à l'exception notoire de la papille dermique qui sera l'élément-clé de la future régénération.

La phase terminale ou phase télogène, qui dure quelques mois, correspond à une phase de repos du follicule à la fin de laquelle le cheveu finit par tomber. A la fin de cette période de repos, un nouveau follicule est régénéré et un nouveau cycle démarre.

Il est par ailleurs été bien décrit que les mécanismes de différenciation des kératinocytes de l'épiderme et du follicule de cheveu sont sensiblement différents. Ainsi, il est connu que les kératines de la tige pilaire représente une famille de kératines distincte de celle exprimée dans l'épiderme (Langbein et al., 2001, J. Biol. Chem. 276), ainsi la kératine K6irs (Porter et al., 2001, Br. J. Dermatol. 145: 558-568) est exprimée dans la gaine interne du follicule pileux mais pas dans l'épiderme, alors que les marqueurs de différenciation épidermiques, tels les kératines K1 et K10, ne sont pas exprimés dans le follicule pileux (Lenoir et al., 1988, Dev. Biol. 130: 610-620).

La chute ou perte naturelle des cheveux peut être estimée, en moyenne, à une centaine de cheveux par jour pour un état physiologique normal. Ce processus de renouvellement permanent peut être perturbé par de nombreux facteurs extrinsèques et intrinsèques, entraînant une perte importante, temporaire ou définitive, des cheveux que l'on regroupe sous le terme générique d'alopécie. Ainsi, des perturbations de la microcirculation ont été observées dans la peau alopécique. Parallèlement, dans les zones alopéciques, une fibrose périfolliculaire s'installe, les follicules se miniaturisent cycle après cycle et progressivement, la vascularisation des bulbes s'amoindrie.

Par ailleurs, le processus de renouvellement physiologique permanent des cheveux est soumis au vieillissement. Si le signe le plus visible du vieillissement du cheveu est le blanchiment, la qualité de l'environnement biologique du follicule du cheveu est également affectée. Parmi les manifestations de vieillissement du cheveu, on observe une moindre synthèse des protéines de la matrice extracellulaire (collagène, laminine, fibronectine), provoquant une perte d'élasticité et de tonicité du tissu sous-cutané. Il y a également une diminution de la cohérence et de l'organisation des follicules de cheveu, une diminution de la durée de la phase anagène et un allongement de la durée de la phase télogène (Courtois et al., 1995, Br. J. dermatol., 132 :86-93). Les cheveux perdent en effet de leur élasticité, ils sont plus fins donc plus fragiles. Au niveau de l'ensemble du cuir chevelu, le vieillissement se manifeste par une diminution de la densité capillaire et par la diminution progressive du diamètre des follicules, donnant à la chevelure un aspect plus pauvre, plus clairsemé (Pelfini, C. et al., J. Méd. Esth. Et Chir. Derm.1987 ; Birch MP et al. Br. J. Dermatol 2001;144:297-304).

Indépendamment du vieillissement intrinsèque, des altérations du cheveu ou du follicule de cheveu peuvent se produire lors d'agressions extérieures. En effet, si le cheveu est d'une remarquable stabilité, certains facteurs extérieurs, tels que le soleil, responsable du photo-vieillissement, les radicaux libres, la pollution ou encore certains traitements inappropriés peuvent aboutir à une détérioration prématurée de la structure des cheveux et à un épuisement des follicules.

On entend par l'expression « agression extérieure », les agressions que peut produire l'environnement. A titre d'exemple, on peut citer des agressions telles que la pollution, les UV, ou encore les produits à caractère irritant tels que les tensioactifs trop détergents, les colorations et décolorations, permanentes ou défrisages trop fréquents, les agressions mécaniques, telles que le frottement des vêtements, les coiffures provoquant des étirements répétés, le brossage trop intense, les brushings, le séchage à l'air trop chaud. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldéhyde), ou bien encore la fumée de cigarette. Ces agressions extérieures aboutissent à une altération de la structure externe du cheveu et de ses propriétés mécaniques, mais peuvent aussi affecter le follicule de cheveu et provoquer un vieillissement prématuré.

L'industrie cosmétique ou pharmaceutique recherche toujours des compositions permettant de supprimer ou de réduire la perte ou de stimuler la croissance des cheveux. Comme molécule connue, on peut citer le 2,4-diamino 6-piperidinopyrimidine 3-oxyde ou "Minoxidil" (brevets US 4 139 619 et US 4 596 812). Un composé connu pour maintenir la vascularisation périfolliculaire est le 4- vérapamil, décrit comme étant actif dans le traitement de la chute des cheveux, en particulier par leurs effets sur la microcirculation (JP 88/062680).

En outre, il existe d'autres brevets décrivant l'utilisation de vasodilatateurs pour application topique, destinés à stimuler la croissance des cheveux en agissant sur la microcirculation du cuir chevelu (EP 327 263). Un autre document brevet de Shiseido (JP 07316023) décrit également l'utilisation de l'arginine et de ses dérivés dans le traitement de l'alopécie. Toutefois, certains produits disponibles présentent des effets secondaires, comme c'est le cas pour le Minoxidil, ou ont une efficacité relative et circonscrite à la durée du traitement. Aussi, il subsiste le besoin d'une nouvelle composition physiologiquement acceptable pour favoriser la croissance des cheveux et/ou réduire leur chute, présentant une action rapide et durable dans le temps.

Des extraits peptidiques de levure ont précédemment été décrits pour leurs propriétés d'hydratation de la fibre capillaire pour une utilisation dans des compositions à rincer pour les cheveux (EP0695801).

D'autre part, des extraits peptidiques de levure ont précédemment été décrits pour leurs effets sur la peau (FR 2904 552, FR 2887 772). Les inventeurs ont maintenant mis en évidence que de tels hydrolysats peptidiques de levure avaient une activité sur le renforcement de la structure du follicule de cheveu et une amélioration de la santé du cheveu. Il a en particulier été démontré que l'hydrolysat peptidique, lorsqu'il est appliqué sur le cheveu, augmente la différenciation des cellules épithéliales de la gaine externe du follicule, améliore la vascularisation du follicule et augmente l'expression des protéines de la matrice extracellulaire.

### Exposé de l'invention

Le premier objet de l'invention est l'utilisation non thérapeutique d'une composition telle que définie dans les revendications comprenant au moins un hydrolysat peptidique de levure (*Saccharomyces cerevisiae*), en tant qu'agent actif, pour renforcer le cheveu et améliorer la santé du cheveu.

Les fibres kératiniques humaines auxquelles s'applique l'invention sont notamment les cheveux, les sourcils, les cils, les poils de barbe, de moustache, les poils pubiens et les ongles. Plus spécialement, l'invention s'applique aux cheveux et/ou aux cils humains.

On entend par « hydrolysat peptidique », un mélange de composés majoritairement représentés par des peptides ou des oligopeptides. Selon l'invention, on utilisera indifféremment les termes « hydrolysat peptidique » ou « agent actif ».

On entend par « agent actif capable de renforcer le cheveu et d'améliorer la santé du cheveu », tout hydrolysat peptidique issu de levure, capable d'augmenter l'expression des kératines K14 et K17, d'augmenter l'expression de protéines de la matrice extracellulaire telle que le collagène I et d'augmenter l'expression de protéines de la lame basale telle que le collagène IV.

On entend également que l'agent actif est capable d'augmenter l'expression de protéines présentes dans la paroi des vaisseaux sanguins présents dans le follicule, tels que la protéine CD34 et le collagène IV.

L'agent actif utilisé selon l'invention peut être obtenu par extraction de protéines de levure, suivie d'une hydrolyse contrôlée qui libère des composés de nature peptidique.

On entend par « composés de nature peptidique », les fragments de protéines et les peptides présents dans l'hydrolysat peptidique. L'utilisation d'hydrolysats peptidiques, et en particulier d'hydrolysats peptidiques de bas poids moléculaires, présente de nombreux avantages en cosmétique. Outre le fait de générer des composés de nature peptidique qui ne préexistaient pas dans le mélange protéique de départ, l'hydrolyse et la purification permettent d'obtenir des mélanges plus stables, plus facilement standardisables et ne provoquant pas de réactions allergiques en dermato-cosmétique.

L'hydrolysat de levure du genre Saccharomyces doit s'entendre comme un hydrolysat de levure appartenant au genre Saccharomyces. Bien entendu l'hydrolysat peut être préparé à partir de levure d'au moins l'une quelconque des nombreuses variétés et espèces appartenant au genre Saccharomyces. Selon un mode de réalisation préférée, ledit agent actif provient de l'hydrolyse de protéines de levure de l'espèce *Saccharomyces cerevisiae.*

De très nombreuses protéines de levure sont susceptibles de contenir des peptides bioactifs au sein de leur structure. L'hydrolyse ménagée permet de dégager ces composés de nature peptidique. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les composés de nature peptidique ensuite.

Selon une méthode de réalisation de l'invention actuellement préférée, l'hydrolysat de levure est un extrait aqueux. Par extrait aqueux, on entend tout ensemble de composés solubles dans l'eau ou dans tout solvant constitué totalement ou partiellement d'eau. Les extraits selon l'invention sont notamment des extraits aqueux purifiés. On peut en particulier citer des solvants aqueux. Par solvant aqueux, on entend tout solvant constitué totalement ou partiellement d'eau. On peut citer l'eau elle-même, le glycérol, les solvants hydro-alcooliques en toutes proportions ou encore les solvants constitués d'eau et d'un composé comme le propylène glycol ou le butylène glycol en toutes proportions. Cet hydrolysat peut être obtenu par dissolution dans l'eau, l'alcool ou l'éther, puis par concentration de cette solution en faisant intervenir l'évaporation ou la distillation.

Toute méthode d'extraction ou de purification connue de l'homme du métier peut être utilisée pour préparer l'hydrolysat. Ainsi, dans une première étape, les levures sont mises en culture de manière classique dans un milieu adapté à leur développement, de préférence en présence de lactose. Elles sont récoltées par centrifugation puis mises en suspension dans une solution tampon, préférentiellement un tampon phosphate. Dans une seconde étape, ces cellules sont éclatées à l'aide d'une presse de french ou à l'aide d'un broyeur à bille, la majorité des composants membranaires insolubles étant écartés par centrifugation ou par filtration.

Un filtrat riche en protéines peut être alors recueilli et remis en solution. Une fraction, riche en composés de nature peptidique, peut alors être isolée par précipitation en milieu alcoolique ou par une solution saline. Les composants solubles et les acides nucléiques sont ainsi écartés. Selon une variante du procédé d'obtention de l'hydrolysat selon l'invention, une étape de dialyse ainsi qu'une étape d'hydrolyse par un cocktail de protéases peuvent être ajoutées afin d'obtenir une fraction riche en composés de nature peptidique.

Une étape supplémentaire de purification par un procédé de type chromatographique peut être envisagée.

Selon un autre mode de réalisation de l'hydrolysat de levure utilisé selon l'invention, l'étape d'hydrolyse peut être réalisée directement sur les levures récoltées après centrifugation ou sur les fractions obtenues après éclatement cellulaire. Des étapes de filtrations et de stérilisation sont par la suite réalisées.

On procède à une phase de dilution dans de l'eau ou dans tout mélange contenant de l'eau, puis cette dilution est stérilisée par ultrafiltration afin d'obtenir un hydrolysat peptidique caractérisé par une concentration en protéines de 30 et 70 % du poids total de l'extrait sec, plus particulièrement cette concentration est de 40 et 50 % du poids total de l'extrait sec. Les solvants utilisés sont physiologiquement acceptables et classiquement utilisés par l'homme du métier, choisis parmi le glycérol, l'éthanol, le propanediol, le butylène glycol, le dipropylène glycol, les diglycols éthoxylés ou propoxylés, les polyols cycliques, ou tout mélange de ces solvants.

Ainsi, l'agent actif utilisé selon l'invention est avantageusement solubilisé dans un ou plusieurs solvants physiologiquement acceptables, tels que l'eau, le glycérol, l'éthanol, le propanediol, le butylène glycol, le dipropylène glycol, les diglycols éthoxylés ou propoxylés, les polyols cycliques ou tout mélange de ces solvants. L'agent actif dilué est ensuite stérilisé par ultrafiltration.

Après cette étape de dilution, l'agent actif peut être encapsulé ou inclus dans un vecteur cosmétique ou pharmaceutique tels que les liposomes ou toute autre microcapsule utilisée dans le domaine de la cosmétique ou adsorbé sur des polymères organiques poudreux, des supports minéraux comme les talcs et bentonites.

Selon un mode de réalisation avantageux, l'agent actif est présent dans les compositions de l'invention à une concentration comprise entre 0,001 % et 5 % environ, et préférentiellement à une concentration comprise entre 0,01 % et 1 % environ par rapport au poids total de la composition finale.

Les compositions utilisables selon l'invention peuvent se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions, ou encore de poudres. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la zone à traiter sous forme d'aérosol. Elles peuvent être utilisées comme produit de soin et/ou comme produit de maquillage de la peau.

L'ensemble de ces compositions comprennent, en outre, tout additif communément utilisé dans le domaine d'application envisagé ainsi que les adjuvants nécessaires à leur formulation, tels que des co-solvants (éthanol, glycérol, alcool benzylique, humectants...), des épaississants, des diluants, des émulsionnants, des antioxydants, des colorants, des filtres solaires, des pigments, des charges, des conservateurs, des parfums, des absorbeurs d'odeur, des huiles essentielles, des oligo-éléments, des acides gras essentiels, des tensioactifs, des polymères filmogènes, des filtres chimiques ou minéraux, des agents hydratants ou des eaux thermales etc. On peut par exemple citer des polymères hydrosolubles de type polymère naturel, tels que les polysaccharides, ou polypeptides, des dérivés cellulosiques de type methylcellulose ou hydroxypropylcellulose, ou encore des polymères synthétiques, polaxamères, carbomères, PVA ou PVP et notamment les polymères vendus par la société ISP.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à une concentration allant de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Les compositions utilisables selon l'invention peuvent en particulier consister en un shampooing, un après-shampooing, une lotion traitante pré- ou post- traitement agressif pour les cheveux, une crème ou un gel coiffant, une lotion restructurante pour les cheveux, un masque, etc. La composition peut également se présenter sous forme de mascara pour une application sur les cils, les sourcils ou les cheveux.

Par ailleurs l'agent actif peut être utilisé seul ou bien en association avec d'autres agents actifs.

Avantageusement, les compositions utilisables selon l'invention contiennent, en outre, au moins un autre agent actif protecteur ou qui améliore la pousse et/ou la santé des cheveux. On peut citer, de manière non limitative, les ingrédients suivants : des vitamines, des agents anti-radicalaires, anti-UV, d'autres extraits peptidiques végétaux, le minoxidil, des esters de l'acide nicotinique, des agents anti-inflammatoires, de l'acide rétinoïque ou ses dérivés, du rétinol, des inhibiteurs de la 5 alpha-réductase ou des composés peptidiques issus de la synthèse chimique.

La composition utilisable selon l'invention pourra être appliquée par toute voie appropriée, notamment orale, parentérale ou topique, et la formulation des compositions sera adaptée par l'homme du métier, en particulier pour des compositions cosmétiques ou dermatologiques.

Avantageusement, les compositions sont destinées à une administration par voie topique. Ces compositions doivent donc contenir un milieu physiologiquement acceptable, c'est-à-dire compatible avec la peau et les phanères, et couvrent toutes les formes cosmétiques ou dermatologiques.

On entend par « application topique », le fait d'appliquer ou d'étaler l'agent actif selon l'invention, ou une composition le contenant, à la surface de la peau ou d'une muqueuse. On entend par « physiologiquement acceptable », que l'hydrolysat peptidique selon l'invention, ou une composition le contenant, est approprié pour entrer en contact avec la peau ou une muqueuse sans provoquer de réactions de toxicité ou d'intolérance.

L'alopécie recouvre un ensemble d'atteintes du follicule pileux ayant pour conséquence finale la perte transitoire ou définitive, partielle ou générale des cheveux. Les hommes comme les femmes peuvent être atteints d'alopécie, mais les zones préférentiellement touchées chez les hommes sont les golfes temporaux ou frontaux, alors que chez les femmes on constate une alopécie diffuse du vertex.

Le deuxième objet de l'invention est l'utilisation d'un hydrolysat peptidique de levure *(Saccharomyces cerevisiae),* en tant qu'agent actif, capable de renforcer les structures du follicule de cheveu. Cette action se caractérise au niveau histologique par une augmentation de la différenciation des cellules épithéliales de la gaine externe du follicule, une stimulation de la vascularisation du follicule de cheveu et une densité accrue de la matrice extracellulaire et de la membrane basale.

Au niveau moléculaire, l'action de l'hydrolysat peptidique de levure (*Saccharomyces cerevisiae*), utilisé selon l'invention se caractérise par l'augmentation de l'expression des kératines K14 et K17.

L'action de l'hydrolysat peptidique de levure (*Saccharomyces cerevisiae*) utilisé selon l'invention se caractérise également l'augmentation de l'expression de la protéine CD34 et du collagène IV dans la paroi des vaisseaux sanguins du follicule de cheveu.

L'action de l'hydrolysat peptidique de levure (*Saccharomyces cerevisiae*), utilisé selon l'invention se caractérise encore par l'augmentation de l'expression du collagène I dans la matrice extracellulaire et l'augmentation de l'expression du collagène IV dans la membrane basale du follicule de cheveu.

Le troisième objet de l'invention concerne un procédé de traitement non-thérapeutique destiné à prévenir ou à lutter contre les agressions extérieures du cheveu, caractérisé en ce que l'on applique topiquement sur la zone à traiter la composition selon l'invention.

Le quatrième objet de l'invention concerne un procédé de traitement non-thérapeutique destiné à restaurer et/ou stimuler la pousse des cheveux, ou lutter contre la chute du cheveu, caractérisé en ce que l'on applique topiquement sur la zone à traiter la composition selon l'invention.

Selon un mode de réalisation particulier, l'invention concerne un procédé de traitement cosmétique destiné à restaurer et/ou stimuler la pousse des cils, ou lutter contre la chute des cils, caractérisé en ce que l'on applique topiquement sur la zone à traiter la composition selon l'invention
Le cinquième objet de l'invention est l'utilisation non-thérapeutique d'un hydrolysat peptidique de levure (*Saccharomyces cerevisiae*), en tant qu'agent actif capable de renforcer et protéger le follicule de cheveu, pour stimuler la pousse du cheveu ou lutter contre la chute du cheveu. Selon cette forme de l'invention, les compositions seront appropriées à une administration orale pour un usage pharmaceutique. Ainsi, les compositions pourront notamment se présenter sous forme de comprimés, capsules, gélules, pâtes à mâcher, poudres à consommer telles quelles ou à mélanger extemporanément avec un liquide, sirop, gels, et toute autre forme connue de l'homme du métier. Ces compositions comprennent, en outre, tout additif communément utilisé dans le domaine d'application envisagé ainsi que les adjuvants nécessaires à leur formulation, tels que des solvants, des épaississants, des diluants, des antioxydants, des conservateurs, d'autres agents actifs pharmaceutiques, des huiles essentielles, des vitamines, des acides gras essentiels, etc.

L'invention concerne encore un procédé de traitement cosmétique destiné à prévenir ou à lutter contre les manifestations du vieillissement et du photo-vieillissement du cheveu, caractérisé en ce que l'on applique topiquement sur la zone à traiter la composition selon l'invention.

L'invention a encore pour objet un procédé de traitement cosmétique destiné à stimuler la pousse des ongles, caractérisé en ce que l'on applique topiquement sur la zone à traiter la composition selon l'invention.

Des modes de réalisation particuliers de ce procédé de traitement cosmétique résultent également de la description précédente. D'autres avantages et caractéristiques de l'invention apparaîtront mieux à la lecture des exemples donnés à titre illustratif et non limitatif.

### Liste des figures

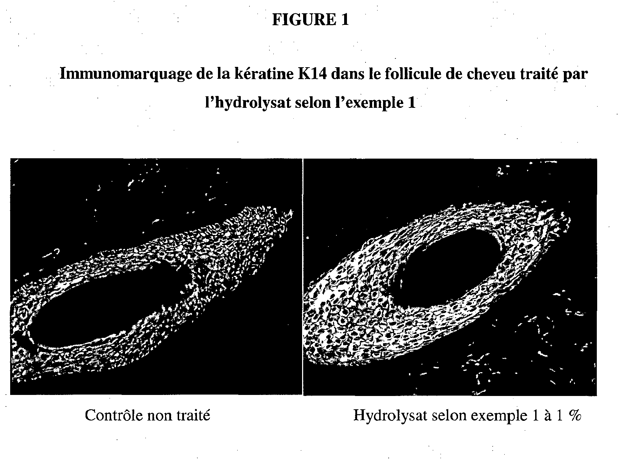

Figure 1 : Immunomarquage de la kératine K14 dans le follicule de cheveu traité par l'hydrolysat selon l'exemple 1.

### Exemple 1 : Préparation d'un hydrolysat peptidique de levure (Saccharomyces cerevisiae)

L'agent actif est obtenu à partir d'un extrait de levure de l'espèce *Saccharomyces cerevisiae.* Les levures sont cultivées dans un milieu adapté à leur développement puis centrifugées pour récupérer une biomasse.

La biomasse est ensuite broyée dans un broyeur à billes. Ensuite, le broyat est remis en suspension dans de l'eau à une concentration de 100 grammes par litre avant une hydrolyse enzymatique entre 40 et 60°C pendant 6 heures. Après hydrolyse, l'extrait est centrifugé puis soumis à une dilution dans un mélange eau-glycérol. L'extrait est ensuite filtré avant stérilisation.

On obtient alors un hydrolysat contenant une quantité de composés de nature protéique et peptidique représentant environ entre 30 et 70 % du poids total de l'extrait sec, plus particulièrement cette quantité est comprise entre 40 et 50 % du poids total de l'extrait sec.

La détermination de la composition en acides aminés de l'agent actif selon l'invention a également été réalisée. Après un dosage des protéines et des peptides par la méthode de Lowry, une hydrolyse acide est réalisée, pour réduire tous les peptides à l'état d'acides aminés libres. Un exemple de composition en acides aminés de l'hydrolysat est donné dans le tableau suivant. Les valeurs sont exprimées en pourcentage d'acides aminés pour 100 g de protéines.

| Acides aminés | % |
|---|---|
| Alanine | 7.4 |
| Acide Aspartique | 12.0 |
| Arginine | 4.6 |
| Acide Glutamique | 15.6 |
| Glycine | 5.5 |
| Histidine | 2.8 |
| Isoleucine | 4.6 |
| Leucine | 9.2 |
| Lysine | 8.3 |
| Phénylalanine | 5.5 |
| Proline | 4.6 |
| Serine | 6.5 |
| Thréonine | 5.5 |
| Tyrosine | 4.6 |
| valine | 6.5 |
| Trytophane | ND |

### Exemple 2 : Mise en évidence de la stimulation de la différenciation des cellules épithéliales de la gaine externe du cheveu par l'hydrolysat selon l'exemple 1

Le but de cette étude est de déterminer l'influence de l'hydrolysat selon l'exemple 1 sur la différenciation des cellules épithéliales de la gaine externe du cheveu. Pour cela, des marqueurs de la différenciation des kératinocytes ont été étudiés. La kératine K14 est un marqueur précoce de la différenciation, exprimé dans le compartiment prolifératif de la gaine épithéliale externe du follicule de cheveu. La kératine K17 est un marqueur de différenciation tardif, étroitement associé à la force de la tige pilaire.

Protocole : Des biopsies de peaux de 6 mm de diamètre provenant de lifting et contenant des follicules de cheveu sont mis en culture sur des inserts dans un milieu WILLIAM E., puis traitées pendant 48 heures par l'hydrolysat selon l'exemple 1 à 1 %. Des contrôles non traités sont également réalisés. A la fin de l'expérience, les biopsies sont mises en cassette et plongées dans un mélange de formol à 10 % pendant 2 heures dans un appareil automatisé (VIP). L'enrobage dans la paraffine est préparé par une série de bains d'alcool (à concentration et temps croissants), suivie de 2 bains de xylène et enfin d'un bain de paraffine. La durée totale de cette série d'opérations est d'une douzaine d'heures. Les biopsies incluses dans la paraffine sont ensuite coupées à 4 µm par un microtome et montées sur des lames. Les lames sont déparaffinées, réhydratées puis soumises à un immunomarquage par un anticorps monoclonal dirigé contre la kératine K14 (Abcam) ou un anticorps monoclonal dirigé contre la kératine K17 (Abcam). puis d'un anticorps secondaire adapté, couplé à un marqueur fluorescent. Les coupes de peau sont alors examinées au microscope à Epi-fluorescence (Nikon Eclipse E 80i microscope).

Résultats : Sur les coupes de follicule de cheveu traitées par l'hydrolysat selon l'exemple 1 et marquées pour la kératine K14 ou la kératine K17, on observe une fluorescence beaucoup plus intense des cellules de la gaine épithéliale externe en comparaison avec le contrôle non traité.

A l'aide d'un logiciel de quantification de la fluorescence, on peut mesurer une augmentation de 44 % pour la kératine K14 et de 44% pour la kératine K17.

Conclusions : L'hydrolysat selon l'exemple 1 augmente la différenciation des kératinocytes, en particulier de la kératine K17 étroitement associée à la résistance de la tige pilaire. L'hydrolysat selon l'exemple 1 augmente la cohésion de la gaine épithéliale externe et améliore la conformation de la gaine épithéliale interne.

Dans son ensemble, cette étude démontre que l'agent actif selon l'invention renforce la structure du cheveu.

### Exemple 3 : Mise en évidence de l'effet stimulant de l'hydrolysat selon l'exemple 1 sur la vascularisation du follicule de cheveu

Le but de cette étude est de déterminer l'influence de l'hydrolysat selon l'exemple 1 sur les vaisseaux sanguins du follicule de cheveu. Pour cela, l'expression de marqueurs de la paroi des vaisseaux sanguins a été étudiée.

Protocole : Les cultures et les inclusions en paraffine sont réalisées selon le même protocole que dans l'exemple 2. Les lames sont déparaffinées, réhydratées, soumises à une étape de démasquage puis à un immunomarquage par un anticorps monoclonal dirigé contre le collagène IV (Chemicon) ou contre la protéine CD34 (Novocastra), puis d'un anticorps secondaire adapté, couplé à un marqueur fluorescent. Les coupes de peau sont alors examinées au microscope à Epi-fluorescence (Nikon Eclipse E 80i microscope).

Résultats : Sur les coupes de follicule de cheveu traitées par l'hydrolysat selon l'exemple 1, et dont le collagène IV a été immunomarqué, on observe une fluorescence plus intense de la lame basale et de la paroi des vaisseaux sanguins comparativement au contrôle non traité. A l'aide d'un logiciel de quantification de la fluorescence, on peut mesurer une augmentation de 144 %.

Sur les coupes de follicule de cheveu traitées par l'hydrolysat selon l'exemple 1, et dont la protéine CD34 a été immunomarquée, on observe une fluorescence plus intense de la paroi des vaisseaux sanguins et de la gaine conjonctive comparativement au contrôle non traité.

Conclusions : L'hydrolysat selon l'exemple 1 améliore la circulation sanguine dans le follicule, ce qui a pour conséquence d'augmenter l'apport de nutriment et ainsi d'améliorer la santé du cheveu.

D'autre part, l'hydrolysat selon l'exemple 1 améliore les structures du follicule de cheveu.

### Exemple 4 : Mise en évidence du renforcement des structures matricielles de l'hydrolysat selon l'exemple 1

Le but de cette étude est de déterminer l'influence de l'hydrolysat selon l'exemple 1 sur la matrice extracellulaire et la papille dermique du follicule. Pour cela l'expression de l'une des principales protéines de la matrice a été étudiée.

Protocole : Les cultures et les inclusions en paraffine sont réalisées selon le même protocole que dans l'exemple 2. Les lames sont déparaffinées, réhydratées, soumises à une étape de démasquage puis à un immunomarquage par un anticorps polyclonal dirigé contre le collagène I (Tebu-Rockland), puis d'un anticorps secondaire adapté, couplé à un marqueur fluorescent. Les coupes de peau sont alors examinées au microscope à Epi-fluorescence (Nikon Eclipse E 80i microscope).

Résultats : Sur les coupes de follicule de cheveu traitées par l'hydrolysat selon l'exemple 1, et dont le collagène I a été immunomarqué, on observe une fluorescence plus intense de la gaine conjonctive et de la papille dermique comparativement au contrôle non traité.

Conclusions : L'hydrolysat selon l'exemple 1 renforce la partie dermique entourant le follicule et assure ainsi une meilleure cohésion et une meilleure protection de la structure folliculaire du cheveu.

### Exemple 5 : Préparation de compositions

### 1 - Soin nourrissant pour cheveu et cuir chevelu

Appliquer le produit sur le cuir chevelu humide. Masser pour répartir uniformément le produit. Renforce les cheveux tout en les rendant lisses et faciles à coiffer.

| **Formulation** | | **1** | **2** | |
|---|---|---|---|---|
| **Nom INCI** | **Nom commercial** | **% massique** | **% massique** | **Fournisseur** |
| Phase A | | | | |
| Deionized Water | - | Q.S. | Q.S. | |
| Aminomethyl Propanol | AMP-95 | 0,05 | 0,05 | |
| Acrylic Acid/VP Crosspolymer | UltraThix™ P-100 | 0,85 | 0,85 | ISP |
| Phase B | | | | |
| Gycerol Dilaurate | Emulsynt™ GDL | 0,50 | 0,50 | ISP |
| Jojoba Seed Oil | - | 2,00 | 2,00 | Lipo |
| Cetearyl Alcohol | - | 2,00 | 2,00 | Rita |
| Phase C | | | | |
| Cyclopentasiloxane | SiTec™ CM040 | 0,50 | 1,00 | ISP |
| Phase D | | | | |
| VP/DMAPA Acrylates Copolymer | Styleze® CC-10 | 3,00 | 3,00 | ISP |
| Water | | 20,00 | 20,00 | |
| Aminomethyl Propanol | | 0,37 | 0,37 | |
| Phase E | | | | |
| Diazolidinyl Urea (and) Methylparaben (and) Propylene Glycol | Germaben® M | 0,75 | 0,75 | ISP |
| | Hydrolysat selon l'exemple 1 | 0,50 | 1,00 | ISP |
| Total | | 100,00 | 100,00 | |

Mettre l'eau et l'AMP-95 dans un récipient sous agitation. Ajouter l'UltraThix™ P-100 dans l'eau sous agitation vigoureuse et maintenir sous agitation pendant 30 minutes. Chauffer la phase A à 65 °C. Chauffer les ingrédients de la phase B à 65 °C puis les mélanger. Ajouter à la phase B et mélanger soigneusement. Refroidir à 35 °C. Ajouter la phase C au mélange principal et mélanger jusqu'à obtenir un aspect uniforme.

Indépendamment, mélanger les ingrédients de la phase D, jusqu'à obtenir un aspect uniforme. Ajouter le Germaben ®M (Phase E) et mélanger jusqu'à obtenir un aspect uniforme. Ajouter l'hydrolysat selon l'exemple 1 et agiter jusqu'à obtenir un aspect uniforme.

### 2 - Sérum pour la pousse des cheveux :

Appliquer le produit sur le cuir chevelu humide. Masser pour répartir uniformément le produit. Favorise la pousse ou repousse des cheveux et rend la chevelure plus nerveuse.

| **Formulation** | | **1** | **2** | |
|---|---|---|---|---|
| **Nom INCI** | **Nom commercial** | **% massique** | **% massique** | **Fournisseur** |
| Water | | Q.S. | Q.S. | |
| Hydroxyethylcellulose | Natrosol 250HHR | 0,35 | 0,50 | Hercules/Aqualon |
| Disodium EDTA | Dissolvine NA-2S | 0,05 | 0,05 | Akzo Nobel |
| VP/DMAPA Acrylates Copolymer | Styleze® CC-10 | 5,00 | 5,00 | ISP |
| Quaternium-26 | Ceraphyl® 65 | 1,00 | 1,00 | ISP |
| Panthenol | Ritapan DL | 0,15 | 0,15 | RITA |
| Propylene Glycol (and) Diazolidinyl Urea (and) Iodopropynyl Butylcarbamate | Liquid Germall® Plus | 0,50 | 0,50 | ISP |
| | Hydrolysat selon l'exemple 1 | 1,00 | 1,00 | ISP |
| Total | | 100,00 | 100,00 | |

Disperser le Natrosol 250HHR et le Disodium EDTA dans l'eau sous agitation. Chauffer à 50-60 °C, et agiter jusqu'à obtenir un aspect uniforme. Ajouter le Styleze® CC-10, et agiter jusqu'à obtenir un aspect uniforme. Laisser refroidir à température ambiante et ajouter les ingrédients dans l'ordre de la liste en agitant jusqu'à obtenir un aspect uniforme entre chaque ajout.

### 3 - Mousse traitante non aérosol :

Appliquer le produit sur le cuir chevelu humide. Masser pour répartir uniformément le produit. Renforce la vitalité et la santé des cheveux et favorise une tenue durable de la coiffure, en particulier en atmosphère humide.

| **Formulation** | | **1** | **2** | |
|---|---|---|---|---|
| **Nom INCI** | **Nom commercial** | **% massique** | **% massique** | **Fournisseur** |
| Water | | Q.S. | Q.S. | |
| PEG-45M | POLYOX N-750 | 0,075 | 0,075 | Dow |
| Polyquaternium-55 (20%) | Styleze® W | 5,00 | 5,00 | ISP |
| Propylene Glycol (and) Diazolidinyl Urea (and) Iodopropynyl Butylcarbamate | Liquid Germall® Plus | 0,50 | 0,50 | ISP |
| PEG/PPG-25/25 Dimethicone | SiTec™ DMC 6031 | 1,00 | - | ISP |
| Palmitamidopropyltrimonium Chloride | Varisoft PATC | - | 0,80 | Degussa |
| | Hydrolysat selon l'exemple 1 | 1,00 | 1,00 | ISP |
| Total | | 100,00 | 100,00 | |

Mette de l'eau dans un récipient convenable, et agiter vigoureusement pour créer un vortex. Disperser le Polyox dans le vortex et agiter jusqu'à complète dissolution. Ajouter le Styleze W-20 et agiter jusqu'à obtenir un aspect uniforme. Ajouter ensuite les ingrédients dans l'ordre de la liste en agitant jusqu'à obtenir un aspect uniforme entre chaque ajout.

## Revendications

1. Utilisation non-thérapeutique d'une composition comprenant au moins un hydrolysat peptidique de levure (*Saccharomyces cerevisiae*) ayant une concentration en composés de nature peptidique comprise entre 30 et 70% du poids total de l'extrait sec, obtenu après éclatement cellulaire et élimination de la majorité des composants membranaires insolubles, en tant qu'agent actif pour renforcer le cheveu et améliorer la santé du cheveu, pour stimuler la pousse des cheveux et/ou des cils humains ou prévenir la chute des cheveux et/ou des cils humains.

2. Utilisation selon la revendication 1, **caractérisée en ce que** l'hydrolysat peptidique a une concentration en composés de nature peptidique comprise entre 40 et 50 % du poids total de l'extrait sec.

3. Utilisation selon l'une des revendications précédentes, **caractérisée en ce que** l'hydrolysat peptidique est solubilisé dans un ou plusieurs solvants physiologiquement acceptables, choisis parmi l'eau, le glycérol, l'éthanol, le propanediol, le butylène glycol, le dipropylène glycol, les diglycols éthoxylés ou propoxylés, les polyols cycliques ou tout mélange de ces solvants.

4. Utilisation selon l'une des revendications précédentes, **caractérisée en ce que** l'hydrolysat peptidique est utilisé en une quantité représentant de 0,001 % à 5 % du poids total de la composition, et préférentiellement en une quantité représentant de 0,01 % à 1 % du poids total de la composition.

5. Utilisation selon l'une des revendications précédentes, **caractérisée en ce que** la composition se présente sous une forme adaptée à l'application par voie topique comprenant un milieu physiologiquement acceptable.

6. Utilisation selon l'une des revendications précédentes, **caractérisée en ce que** la composition comprend en outre au moins un autre agent actif protecteur ou améliorant la pousse et/ou la santé des cheveux.

7. Utilisation selon l'une des revendications 1 à 3, dans laquelle l'hydrolysat peptidique est un agent actif, pour renforcer les structures du follicule de cheveu.

8. Utilisation selon la revendication 7, **caractérisée en ce que** l'agent actif augmente l'expression de la kératine 14 et de la kératine K17 dans les cellules de la gaine externe du follicule de cheveu.

9. Utilisation selon la revendication 7, **caractérisée en ce que** l'agent actif augmente l'expression du collagène IV et de la protéine CD34 dans la paroi des vaisseaux sanguins du follicule de cheveu.

10. Utilisation selon la revendication 7, **caractérisée en ce que** l'agent actif augmente l'expression du collagène I dans la gaine conjonctive et la papille dermique du follicule de cheveu.

11. Procédé de traitement non-thérapeutique destiné à restaurer et/ou stimuler la pousse du cheveu, ou lutter contre la chute du cheveu, **caractérisé en ce que** l'on applique topiquement sur la zone à traiter une composition telle que définie dans l'une des revendications 1 à 6.

12. Procédé de traitement non-thérapeutique destiné à restaurer et/ou stimuler la pousse des cils, ou lutter contre la chute des cils, **caractérisé en ce que** l'on applique topiquement sur la zone à traiter une composition telle que définie dans l'une des revendications 1 à 6.

## Patentansprüche

1. Nicht-therapeutische Verwendung einer Zusammensetzung, die mindestens ein peptidisches Hydrolysat von Hefe (*Saccharomyces cerevisiae*) umfasst, welches eine Konzentration an Verbindungen von Peptidnatur im Bereich zwischen 30 und 70 % des Gesamtgewichts des Trockenextrakts aufweist, welches nach Zellplatzen und Entfernen des Großteils der nicht löslichen Membranbestandteile erhalten wird, als Wirkstoff, um das Haar zu stärken und die Gesundheit des Haars zu verbessern, um das Wachstum der menschlichen Haare und/oder der Wimpern zu stimulieren oder den Ausfall der menschlichen Haare und/oder der Wimpern zu verhindern.

2. Verwendung nach Anspruch 1, **dadurch gekennzeichnet, dass** das peptidische Hydrolysat eine Konzentration an Verbindungen von Peptidnatur im Bereich zwischen 40 und 50 % des Gesamtgewichts des Trockenextrakts aufweist.

3. Verwendung nach einem der vorstehenden Ansprüche, **dadurch gekennzeichnet, dass** das peptidische Hydrolysat in einem oder mehreren physiologisch verträglichen Lösungsmitteln löslich gemacht ist, ausgewählt aus Wasser, Glycerin, Ethanol, Propandiol, Butylenglykol, Dipropylenglykol, den ethoxylierten oder propoxylierten Diglykolen, den cyclischen Polyolen oder jeder Mischung dieser Lösungsmittel.

4. Verwendung nach einem der vorstehenden Ansprüche, **dadurch gekennzeichnet, dass** das peptidische Hydrolysat in einer Menge verwendet wird, die 0,001 % bis 5 % des Gesamtgewichts der Zusammensetzung ausmacht, und vorzugsweise in einer Menge, die 0,01 % bis 1 % des Gesamtgewichts der Zusammensetzung ausmacht.

5. Verwendung nach einem der vorstehenden Ansprüche, **dadurch gekennzeichnet, dass** sich die Zusammensetzung in einer zur Anwendung auf topischem Wege geeigneten Form darstellt, die ein physiologisch verträgliches Medium umfasst.

6. Verwendung nach einem der vorstehenden Ansprüche, **dadurch gekennzeichnet, dass** die Zusammensetzung weiter mindestens einen weiteren schützenden oder das Wachstum und/oder die Gesundheit der Haare verbessernden Wirkstoff umfasst.

7. Verwendung nach einem der Ansprüche 1 bis 3, wobei das peptidische Hydrolysat ein Wirkstoff ist, um die Strukturen des Haarfollikels zu stärken.

8. Verwendung nach Anspruch 7, **dadurch gekennzeichnet, dass** der Wirkstoff die Expression des Keratins 14 und des Keratins K17 in den Zellen der äußeren Hülle des Haarfollikels steigert.

9. Verwendung nach Anspruch 7, **dadurch gekennzeichnet, dass** der Wirkstoff die Expression des Kollagens IV und des Proteins CD34 in der Wand der Blutgefäße des Haarfollikels steigert.

10. Verwendung nach Anspruch 7, **dadurch gekennzeichnet, dass** der Wirkstoff die Expression des Kollagens I in der Bindehauthülle und der Hautpapille des Haarfollikels steigert.

11. Nicht-therapeutisches Behandlungsverfahren, das dazu vorgesehen ist, das Wachstum des Haars wiederherzustellen und/oder zu stimulieren, oder den Ausfall des Haars zu bekämpfen, **dadurch gekennzeichnet, dass** eine Zusammensetzung wie in einem der Ansprüche 1 bis 6 definiert topisch auf dem zu behandelnden Bereich angewendet wird.

12. Nicht-therapeutisches Behandlungsverfahren, das dazu vorgesehen ist, das Wachstum der Wimpern wiederherzustellen und/oder zu stimulieren, oder den Ausfall der Wimpern zu bekämpfen, **dadurch gekennzeichnet, dass** eine Zusammensetzung wie in einem der Ansprüche 1 bis 6 definiert topisch auf dem zu behandelnden Bereich angewendet wird.

## Claims

1. Non-therapeutic use of a composition comprising at least one yeast (*Saccharomyces cerevisiae*) peptide hydrolysate having a concentration of peptide type compounds between 30 and 70% of the total weight of the dry extract, obtained after cell bursting and removal of the majority of the insoluble membrane components, as an active agent for strengthening hair and improving the health of hair, for stimulating the growth of human hair and/or eyelashes or preventing the loss of human hair and/or eyelashes.

2. Use according to claim 1, **characterised in that** the peptide hydrolysate has a concentration of peptide type compounds between 40 and 50% of the total weight of the dry extract.

3. Use according to one of the preceding claims, **characterised in that** the peptide hydrolysate is solubilised in one or a plurality of physiologically acceptable solvents, chosen from water, glycerol, ethanol, propanediol, butylene glycol, dipropylene glycol, ethoxylated or propoxylated diglycols, cyclic polyols or any mixture of these solvents.

4. Use according to one of the preceding claims, **characterised in that** the peptide hydrolysate is used in a quantity representing from 0.001% to 5% of the total weight of the composition, and preferentially in a quantity representing from 0.01% to 1% of the total weight of the composition.

5. Use according to the preceding claims, **characterised in that** the composition is presented in a form suitable for application by the topical route comprising a physiologically acceptable medium.

6. Use according to one of the preceding claims, **characterised in that** the composition further comprises at least one further protective active agent or active agent improving hair growth and/or health.

7. Use according to one of claims 1 to 3, wherein the peptide hydrolysate is an active agent, for strengthening the structures of the hair follicle.

8. Use according to claim 7, **characterised in that** the active agent increases the expression of keratin 14 and or keratin K17 in the cells of the outer sheath of the hair follicle.

9. Use according to claim 7, **characterised in that** the active agent increases the expression of collagen IV and of CD34 protein in the wall of the blood vessels of the hair follicle.

10. Use according to claim 7, **characterised in that** the active agent increases the expression of collagen I in the connective tissue sheath and the dermal papilla of the hair follicle.

11. Non-therapeutic treatment method intended to restore and/or stimulate hair growth, or combat hair loss, **characterised in that** a composition as defined in one of claims 1 to 6 is applied topically onto the area to be treated.

12. Non-therapeutic treatment method intended to restore and/or stimulate eyelash growth, or combat eyelash loss, **characterised in that** a composition as defined in one of claims 1 to 6 is applied topically onto the area to be treated.
